# EUROPEAN PATENT SPECIFICATION

(11) **EP 0 766 968 B1**
(45) Date of publication and mention of the grant of the patent: **17.01.2001**
(21) Application number: 95115758.5
(22) Date of filing: 06.10.1995
(51) Int. Cl.: A61K 51/12, A61K 49/00, A61K 47/48

(54) **Diagnostic imaging agent**
Diagnostikum zur Bilderzeugung
Agent pour l'imagerie diagnostique

(43) Date of publication of application: 09.04.1997
(73) Proprietor: NIHON MEDI-PHYSICS CO., LTD., Hyogo (JP)
(72) Inventor: Hashiguchi, Yuji, c/o Nihon Medi-Physics Co., Ltd., Chiba, 299-02 (JP); Kubomura, Kan, Chiba, Chiba, 261 (JP); Kamada, Toshihiro, Nubo-Shizuichi 403, 42, Kyoto, Kyoto, 601-11 (JP); Seri, Shigemi, Chiba, 299-01 (JP); Iwai, Kumiko, Chiba, 290 (JP)
(74) Representative: von Kreisler, Alek, Dipl.-Chem.

(56) References cited:
- EP-A- 0 357 163
- DATABASE CHEMABS CHEMICAL ABSTRACTS SERVICE, COLUMBUS, OHIO, US AN=115:274850, KOBAYASHI, MIKIHIKO ET AL 'Application of periodate oxidized glucans to biochemical reactions' & J. CARBOHYDR. CHEM. (1991), 10(4), 635-44 CODEN: JCACDM;ISSN: 0732-8303, 1991
- AGRIC. BIOL. CHEM. (1988), 52(11), 2695-702 CODEN: ABCHA6;ISSN: 0002-1369, 1988 pages 2695-2702, KOBAYASHI, MIKIHIKO ET AL 'Cyclodextrin - dialdehyde prepared by periodate oxidation'

## Description

### FIELD OF THE INVENTION

The present invention relates to a diagnostic imaging (hereinafter sometimes abbreviated as MRI) agent, in particular, to a diagnostic imaging agent, useful for nuclear magnetic resonance image diagnosis, X-ray image diagnosis and radiation image diagnosis, containing a metal complex compound having, as its skeleton, an oxidation-cleaved compound of cyclodextrin such as α-cyclodextrin, β-cyclodextrin, γ-cyclodextrin and the like.

### BACKGROUND OF THE INVENTION

(Diethylenetriaminepentaacetic acid)gadolinate (hereinafter abbreviated as "DTPA-Gd") [JP-A 58-29718] which is widely used as a nuclear magnetic resonance diagnostic imaging agent is a representative example of mono-metal complexes and effectiveness thereof as a diagnostic imaging agent in the brain or spinal cord regions has almost been established. However, since DTPA-Gd is complexed, the relaxivity is lower than that of Gd itself. Therefore, there is a need for compensating for the lowered relaxivity with the increased dosage or the like. In addition, DTPA-Gd is rapidly excreted into the urine after administration [Hiroki Yoshikawa et al., Gazoshindan, 6, pages 959-969 (1986)], and this is very disadvantageous for imaging of several parts of the body by reflecting them in blood stream (blood vessel distribution, blood stream distribution, distribution volume, permeation and the like in a lesion) with a single injection of the pharmaceutical.

In order to solve these problems, paramagnetic metal complex compounds obtained by introducing a plurality of paramagnetic metal complexes into a carrier polymer have been developed. However, when a polymeric compound having the molecular weight of not less than several thousands is used as a carrier, there arise problems such as unnecessarily longer retention in the blood, retention in a living body, antigenicity and the like. Therefore, the prior paramagnetic complex compounds are not necessarily satisfactory. For that reason, a nuclear magnetic resonance imaging agent obtained by coupling a paramagnetic metal complex to an oligosaccharide and polysaccharide having the relatively low molecular weight have been developed [JP-A 5-25059]. However, there still remain the problems that longer total time for synthesis is required because of purification upon production of an aldehyde group by oxidation-cleavage of constituent D-glucose, and the complicated procedures such as dialysis, ion exchange and the like are required for removing an excess oxidizing agent.

### OBJECTS OF THE INVENTION

The main object of the present invention is to provide a diagnostic imaging agent, useful for nuclear magnetic resonance image diagnosis, X-ray diagnosis and radiation diagnosis, which can be easily prepared without purification procedures upon preparation of the diagnostic imaging agent by oxidation-cleavage reaction of saccharide skeleton, the coupling of a metal complex and the like, and which is stable, have good solubility in water and is physiologically acceptable.

This object as well as other objects and advantages of the present invention will become apparent to those skilled in the art from the following description with reference to the accompanying drawing.

### SUMMARY OF THE INVENTION

In order to solve the above problems, the present inventors studied hard and, as the result, we found that when cyclodextrins are oxidation-cleaved for a longer period of time, the peroxidation dose not occur, thereafter a metal complex compound can be easily coupled thereto, and a metal complex compound obtained by using such the compound as a starting material is a diagnostic imaging agent useful for nuclear magnetic resonance image diagnosis, X-ray diagnosis and radiation diagnosis, which is stable, has good solubility in water and is physiologically acceptable.

That is, the present invention provides a diagnostic imaging agent which comprises a compound of (a) a complexing agent having at least one ligand chemically coupled to an aldehyde group of a dialdehyde-cyclodextrin wherein at least one constituent monosaccharide cyclodextrin is oxidation-cleaved, coordinated with (b) at least one metal ion selected from the group consisting of metal ions having the atomic number of 21-29, 31, 32, 37-39, 42-44, 49 and 56-83.

The present invention can provide a useful diagnostic imaging agent comprising a compound wherein a metal ion is coordinated to a complexing agent where a ligand is coupled to a novel and particular compound (dialdehyde-cyclodextrin). The diagnostic imaging agent can be easily prepared without purification procedures upon preparation of the diagnostic imaging agent by oxidation-cleavage reaction of saccharide skeleton, the coupling of a metal complex and the like, and is stable, have good solubility in water and is physiologically acceptable. Therefore, the diagnostic imaging agent is useful for nuclear magnetic resonance image diagnosis, X-ray diagnosis and radiation diagnosis.

### BRIEF EXPLANATION OF DRAWING

Figure 1 is a MRI showing a transverse view of the cancer-carrying region of a rat after administration of a DA-α-CD-DO3MA-Gd solution.

Figure 2 is a MRI showing a transverse view of the cancer-carrying region of a rat after administration of a DA-γ-CD-DO3MA-Gd solution.

### DETAILED DESCRIPTION OF THE INVENTION

Cyclodextrin used in the present invention is a compound obtained by reacting starch with amylase (cyclodextrinase). In the compound, D-glucose molecules are circularly connected via α-1,4 bonding. When the number of constituent glucose molecules is 6, the compound is called α-cyclodextrin, when the number of constituent glucose molecules is 7, the compound is called β-cyclodextrin, and when the number of constituent glucose molecules is 8, the compound is called γ-cyclodextrin.

A dialdehyde-compound of cyclodextrin such as α-cyclodextrin, β-cyclodextrin, γ-cyclodextrin and the like is obtained by oxidation-cleavage of a constituent monosaccharide of D-glucose. For example, α-cyclodextrin is dissolved in water, a small amount of sodium metaperiodate is added thereto in portions under the shielded light while stirring at room temperature, followed by further stirring for 24 hours to obtain dialdehyde-α-cyclodextrin. As an oxidizing agent, periodic acid (HIO₄), potassium periodate (KIO₄) and the like are used in addition to sodium metaperiodate (NaIO₄). The reaction is carried out under the conditions of cyclodextrin concentration (1 mmol/l-30 mmol/l), oxidizing agent concentration (0.02M-0.2M), reaction temperature (5°C-20°C), pH (3-5), and reaction time (1-25 hours). These reaction conditions are selected depending upon the extent of the conversion into dialdehyde by cleavage of constituent monosaccharide cyclodextrin. Usually, it takes about 24 hours to cleave all constituent monosaccharides.

For preparing a diagnostic imaging agent of the present invention, the conversion into dialdehyde is carried out by oxidation-cleavage of saccharides and, thereafter, a bifunctional ligand can be reacted therewith to couple to the saccharide skeleton. Upon this, the saccharide is peroxidized with an oxidizing agent. Upon the coupling reaction of the bifunctional ligand after a step of conversion into dialdehyde, the saccharide is peroxidized by the remaining oxidizing agent. These peroxidations disadvantageously lead to the decomposition of the saccharide, which results in decrease in the purity. For example, it was found that, in the case of oxidation-cleavage of maltopentaose wherein five D-glucose molecules are connected in a linear fashion through α-1,4 bonding, more than necessary amount of sodium metaperiodate had already been consumed at 2 hours after the initiation of reaction and therefore, peroxidation had occurred, while in the case of oxidation-cleavage of α-cyclodextrin, sodium metaperiodate was consumed only at an amount for oxidation-cleaving α-cyclodextrin even at 48 hours after the initiation of reaction. Like this, cyclodextrin can be stably converted into dialdehyde by oxidation-cleavage and, even when an excess amount of sodium metaperiodate remains, a next step of labelling a metal complex compound can be conducted without decomposition of cyclodextrin. Therefore, the complicated purification procedures become unnecessary during the synthesis step and the synthesis procedures can be carried out more simply.

As a complexing agent, a linear or cyclic polyaminocarboxylic acid having an activated amino group as a crosslinking chain reactive with an aldehyde group of the above dialdehyde compound of cyclodextrin and a bifunctional structure which is capable of trapping a metal ion to form a complex is used. Particularly preferably, a bifunctional complexing agent, having an activated amino group, which is a derivative of DTPA (diethylenetriaminepentaacetic acid), DOTA (1,4,7,10-tetraazacyclodocecane-1,4,7,10-tetraacetic acid) or TETA (1,4,8,11-tetraazacyclotetradecane-1,4,8,11-tetraacetic acid) is used. Particularly, there are 1-(p-aminobenzyl)diethylenetriaminepentaacetic acid [Martin, W.B. et al.: Inorg. Chem., 25, pages 2772-2781 (1986)], 2-(p-aminobenzyl)-1,4,7,10-tetraacetic acid [U.S. Patent 4678667], 2-aminobutyl-1,4,7,10-tetraazacyclododecane-1,4,7,10-tetraacetic acid [Parker, D. et al.: Pure & Appl. Chem., 61, pages 1637-1641 (1989)] and the like.

Dialdehyde-cyclodextrin is coupled with the complexing agent according to a known method. As described above, the reaction can be carried out in situ without removing an oxidizing agent used for synthesis of dialdehyde-cyclodextrin. This eliminates the necessity of the complicated purification procedures during the synthesis step, which results in the simpler synthesis procedures. For example, dialdehyde-cyclodextrin is reacted with the complexing agent in an alkaline solution to obtain a compound wherein both of them are coupled through -CH=N-. Alternatively, the complexing agent to which a metal is precomplexed may be used. If necessary, this coupled compound may be reduced to convert -CH=N- into -CH₂-NH-. For example, a metal complex compound wherein 1,4,7,10-tetraazacyclododecane-1-aminoethylcarbamoylmethyl-4,7,10-tris[(R,S)-methylacetic acid] (hereinafter abbreviated as "DO3MA") as a bifunctional ligand is coupled to a dialdehyde compound of α-cyclodextrin (hereinafter abbreviated as "DA-α-CD) and Gd as a metal ion is coordinated therewith can be synthesized according to the following procedures:

Sodium metaperiodate is added to an aqueous solution of α-cyclodextrin to effect an oxidation-cleave, DO3MA wherein Gd is coordinated therewith (hereinafter abbreviated as "DO3MA-Gd) according to the conventional method is added thereto, and triethylamine is added thereto as an accelerator to stir. In order to reduce the resulting Schiff base, sodium borohydride is added to the reaction solution to stir, followed by purification by dialysis to obtain DA-α-CD-DO3MA-Gd. Since the use of cyclodextrin eliminates the necessity of the purification procedures during the reaction, it not only facilitates the synthesis but also improves the yield and, as the result, reduces the cost.

The metal ion used in the present invention is selected from the group consisting of metal ions having the atomic number of 21-29, 31, 32, 37-39, 42-44, 49 and 56-83 depending upon a particular use of image diagnosis. When the diagnostic imaging agent of the present invention is used for nuclear magnetic resonance image diagnosis, the metal ion must be paramagnetic and selected from lanthanide ions having the atomic number of 26 and 57-70, and Gd, Dy, Tb, Ho, Er and Fe being preferable. When the diagnostic imaging agent of the present invention is used for X-ray image diagnosis, the metal ion is selected from the group consisting of lanthanide ions having the atomic number of 57-70 and metal ions having the atomic number of 56, 76, 82 and 83. Among them, Bi, Pb and Os ion are preferable. When used as a radiation diagnostic imaging agent, the metal ion must be radioactive and radioactive metal ions: Co, Cu, Ga, Ge, Sr, Y, Tc, In, Sm, Gd, Yb, Re and Ir ion are suitable. The metal ion to be used may be resulted from a metal per se or an inorganic compound containing such the metal (for example, chloride, oxide and the like). The complexing can be carried out according to a conventional method.

In the metal complex compound thus obtained, at least one complexing agent is chemically coupled to a dialdehyde-compound wherein at least one, preferably, two or more constituent saccharides of α-cyclodextrin, β-cyclodextrin and γ-cyclodextrin are oxidation-cleaved, and a metal ion is coupled thereto by complexation.

An amount of the diagnostic imaging agent of the present invention to be used is selected depending upon a particular use of image diagnosis. For example, when used as a nuclear magnetic resonance imaging agent, the dose of the metal ion is generally 0.005 to 5 mmol/kg, preferably 0.01 to 0.5 mmol/kg. When used as a X-ray diagnostic imaging agent, the dose of the metal ion is 0.01 to 10 mmol/kg, preferably 0.1 to 1 mmol/kg. When used as a radiation diagnostic agent, the dose is 370-18500 MBq radioactivity. Usually, the diagnostic imaging agent of the present invention is administered intravenously and, in some cases, may be administered orally or intra-arterially.

At 1 hour after DA-α-CD-DO3MA-Gd which is the diagnostic imaging agent of the present invention was administered to a cancer-carrying rat, the cancer part was clearly imaged and, thus, in vivo imaging effects and retention in the blood were confirmed. In addition, it was also confirmed that T1 relaxivity (intensity of magnetic field: 6.35T, 25°C) in water was remarkably increased to 8.6 (mM•S)⁻¹, about two times of that of DTPA-Gd.

Retention in the blood of the diagnostic imaging agent of the present invention is in a clinically effective range (half-life period in blood of 0.5-5 hours). This is effective for improving the collection efficacy of proton relaxation effect by the imaging agent, for example, in the case of a lower magnetic field intensity MRI apparatus in which imaging requires longer period of time in comparison with a higher magnetic field intensity MRI apparatus. In addition, the higher imaging effect per unit dosage is advantageous. For example, since the shortening effect of the relaxation time per molecule is predominantly stronger than that of DTPA-Gd, the present diagnostic imaging agent can be used advantageously as a nuclear magnetic resonance imaging agent. In addition, in the case of diagnosis using a lower magnetic field intensity MRI apparatus having the lower collecting efficacy of proton relaxation effect, since the present diagnostic imaging agent has the higher imaging efficacy per unit dosage, the detection effect is improved and a time necessary for imaging can be shortened. Furthermore, when the same imaging effect as that of DTPA-Gd in an apparatus having the same magnetic field intensity is required, the present diagnostic imaging agent can be administered in a smaller dose than that of DTPA-Gd and, therefore, becomes more advantageous from the standpoint of safety. To the contrary, at the same dose, the present diagnostic imaging agent provides much more information about a living body than DTPA-Gd, resulting in the improvement in the clinical usefulness. Therefore, the present invention can provide an imaging agent having appropriate retention in the blood and effectively enhanced efficacy, which matches the magnetic field intensity of a MRI spectrometer or imaging conditions.

Further, since the diagnostic imaging agent of the present invention shows appropriate retention in the blood, a blood vessel distribution image (vascularity) can be evaluated. Therefore, since the diagnostic imaging agent of the present invention can image the blood vessel for recently remarkably advanced MR angiography without particular pulse sequence, the agent is also useful as a transvenous diagnostic imaging agent. Further, since the present diagnostic imaging agent has good solubility in water, the agent as such can be prepared into a solution containing the agent in a high concentration. Accordingly, a solubilizer is not necessarily required upon preparation of the solution. In addition, the present diagnostic imaging agent is a poly-chelates compound and, therefore, can decrease the total molality in the preparation of a solution in comparison with the mono-chelate compound, which results in decrease in the osmotic pressure. These alleviate the load to volume of the circulatory system or body fluid equilibrium upon administration in the living body, which resulting in advantage in the safety.

The diagnostic imaging agent of the present invention is prepared by dissolving in distilled water for injection or a physiologically acceptable aqueous solvent. If necessary, a pH adjusting agent or a solubilizer may be added therein. Examples of the pH adjusting agent are NaOH, HCl and the like. Examples of the solubilizer are tri-N-methylglucamine and the like.

The following Examples further illustrate the present invention in detail but are not to be construed to limit the scope thereof.

The abbreviations used in Examples mean as follows:
DTEN: 1-(p-aminobenzyl)diethylenetriaminepentaacetic acid
DO3MA: 1,4,7,10-tetraazacyclododecane-1-aminoethylcarbamoylmethyl-4,7,10-tris[(R,S)-methylacetic acid]
DA-α-CD: dialdehyde-α-cyclodextrin
DA-γ-CD: dialdehyde-γ-cyclodextrin

The composition was analyzed as follows:
C, H and N were analyzed using elementary analysis, a metal ion was analyzed using induced coupling high frequency plasma analysis. The results are represented by wt%. Wt% of oxygen was obtained by subtracting these wt%'s from 100%. The number of coupled metal ions was calculated using a ratio relative to the content of the metal ion which coordinates to all constituent mono saccharide of each cyclodextrin.

### Example 1

### Oxidation-cleavage of α-cyclodextrin or maltopentaose.

Comparative experiment of oxidation-cleavage was carried out using α-cyclodextrin as a cyclodextrin or maltopentaose as a linear polysaccharide.

α-Cyclodextrin (1.20 g; 1.24 mmol) was dissolved in water (10 ml), followed by stirring at room temperature. Under the shielded light, 0.2N sodium metaperiodate (45 ml) was added thereto in portions, followed by continuous stirring. Separately, maltopentaose (1.10 g; 1.33 mmol) was dissolved in water (10ml) to stir at room temperature. Under the shielded light, 0.2N sodium metaperiodate (55 ml) was added thereto in portions, followed by continuous stirring. Each 0.5 ml aliquot was taken from each reaction solution in course of time, and an amount of consumed periodic acid was determined using Flaury-Lange method [P.F.Flery, J.Lange, J.Pharm., 17, 107, 196 (1933)]. As the result, in the case of α-cyclodextrin reaction solution, an amount of consumed periodic acid was increased in course of time after addition of sodium metaperiodate, a necessary amount for cleaving all constituent saccharides was consumed after 24 hours and, thereafter, no increase in the consumed amount was observed. On the other hand, in the case of maltopentaose reaction solution, an amount of consumed periodic acid was similarly increased and a necessary amount for cleaving all constituent saccharides was consumed at 2 hours after addition. Thereafter, the consumed amount however continued to increase, and about 1.4 times periodic acid was consumed at 24 hours after addition in comparison with at 2 hours after addition. Thus, in the latter case, it was confirmed that peroxidation reaction by excess periodic acid proceeded. The results of experiment are shown in Table 1.

**Table 1**

| Oxidation-cleavage by sodium metaperiodate | | | | | | | | |
|---|---|---|---|---|---|---|---|---|
| | Amount of consumed periodic acid (mmol) | | | | | | | Theoretical amount consumed |
| Reaction time | 15min, | 30min, | 1hr, | 2hr, | 3hr, | 24hr, | 48hr | |
| A | - | 1.29 | 3.00 | 3.71 | 5.18 | 7.44 | 7.44 | 7.44 |
| B | 4.65 | 5.32 | 5.98 | 6.65 | 7.30 | 9.31 | - | 6.65 |
| A: α-cyclodextrin | | | | | | | | |
| B: maltopentaose | | | | | | | | |

### Example 2

### Synthesis of DA-α-CD-DTEN-Gd

α-Cyclodextrin (1.2 g; 1.24 mmol) was dissolved in water (9 ml) to stir at room temperature. Under the shielded light, 0.2N sodium metaperiodate (44 ml) was added thereto in portions to stir for 24 hours. Then, DTEN (2.9 g; 5.9 mmol) was added thereto, followed by addition of triethylamine (0.18 ml). After stirred for 24 hours, sodium borohydride (2.8 g) was added thereto to stir for 24 hours. The reaction solution was adjusted to pH 2 with hydrochloric acid and an aqueous sodium hydroxide solution was added to adjust the solution to neutral. GdCl₃·6H₂O (2.4 g; 6.5 mmol) was added to react while stirring at room temperature, and the white precipitates were removed by concentration, followed by dialysis (fraction molecular weight: 1000) to obtain DA-α-CD-DTEN-Gd (0.74 g).
Elementary analysis: C 38.0 wt%; H 7.2 wt%; N 9.0 wt%.
Gd content: 13.9 wt%, Coupled number of Gd: 4.3

### Example 3

### Synthesis of DA-α-CD-DO3MA-Gd

α-Cyclodextrin (2.70 g; 2.78 mmol) was dissolved in water (20 ml) to stir at room temperature. Under the shielded light, 0.2N sodium metaperiodate (100 ml) was added thereto in portions to stir for 24 hours. Then, DO3MA-Gd (8.68 g; 13.5 mmol) was added thereto, followed by addition of triethylamine (0.40 ml). After stirred for 24 hours, sodium borohydride (6.38 g) was added thereto to stir for 24 hours. The reaction solution was adjusted to pH 2 with hydrochloric acid, an aqueous sodium hydroxide solution was added to adjust the solution to neutral, and white precipitates were removed by concentration, followed by dialysis (fraction molecular weight: 1000) to obtain DA-α-CD-DO3MA-Gd (1.97 g).
Elementary analysis: C 38.2 wt%; H 7.3 wt%; N 9.3 wt%
Gd content: 14.8 wt%, Coupled number of Gd: 4.5

### Example 4

### Synthesis of DA-γ-CD-DO3MA-Gd

Substantially the same manner as that in Example 3 except that γ-cyclodextrin was used instead of α-cyclodextrin afforded DA-γ-CD-DO3MA-Gd (1.68 g).
Elementary analysis: C 38.0 wt%; H 7.3 wt%; N 9.8 wt%
Gd content: 15.5 wt%, Gd binding number: 6.2

### Example 5

### Synthesis of DA-β-CD-DO3MA-Bi

β-Cyclodextrin (0.20 g; 0.18 mmol) was dissolved in water (2 ml) to stir at room temperature. Under the shielded light, 0.2N sodium metaperiodate (8 ml) was added thereto in portions to stir for 24 hours. Then, DO3MA-Bi (1.04 g; 1.5 mmol) was added thereto, followed by addition of triethylamine (0.04 ml). After stirred for 24 hours, sodium borohydride (0.6 g) was added thereto to stir for 24 hours. The reaction solution was adjusted to pH 2 with hydrochloric acid, an aqueous sodium hydroxide solution was added to adjust the solution to neutral, and the white precipitates were removed by concentration, followed by dialysis (fraction molecular weight: 1000) to obtain DA-β-CD-DO3MA-Bi (0.48 g).
Bi content: 17.7 wt%, Coupled number of Bi: 4.3

### Example 6

### Relaxivity (in vitro) of DA-α-CD-DO3MA-Gd and DA-γ-CD-DO3MA-Gd

DA-α-CD-DO3MA-Gd or DA-γ-CD-DO3MA-Gd was dissolved in distilled water in terms of Gd concentration: 5, 2.5, 1.25 and 0.625 mM, respectively. Relaxation time of each compound at 37°C (T1 and T2; msec) was determined using NMR (0.5T and 1.5T). T1 relaxivity and T2 relaxivity (R1 and R2, (mM•S)⁻¹, respectively) were calculated from relaxation time values. The results are shown in Table 2. The values are per one Gd molecule. DA-α-CD-DO3MA-Gd and DA-γ-CD-DO3MA-Gd have good relaxation effect in vitro, which is predominantly higher than that of DTPA-Gd (also shown in Table 2), of a mono-chelate complex, measured according to the same method. Thus, the efficacy of the compound in the present invention was confirmed.

**Table 2**

| Relaxivity (in vitro) of DA-α-CD-DO3MA-Gd and DA-γ-CD-DO3MA-Gd | | |
|---|---|---|
| Compound | R1 (mM•S)⁻¹ | R2 (mM•S)⁻¹ |
| DA-α-CD-DO3MA-Gd | 8.6 | 10.8 |
| DA-γ-CD-DO3MA-Gd | 8.4 | 10.8 |
| DTPA-Gd | 3.7 | 4.5 |

### Example 7

### Preparation of a 0.5 M solution of DA-α-CD-DO3MA-Gd or DA-γ-CD-DO3MA-Gd

DA-α-CD-DO3MA-Gd (1.0 g) was weighed and water for injection was added thereto at an appropriate amount to obtain a 0.5M (Gd concentration) solution of DA-α-CD-DO3MA-Gd.

DA-γ-CD-DO3MA-Gd (1.0 g) was weighed and water for injection was added thereto at an appropriate amount to obtain a 0.5M (Gd concentration) solution of DA-γ-CD-DO3MA-Gd. Both compounds could be prepared into a solution having the higher neccesary concentration without the use of a solubilizer.

### Example 8

Imaging effect (in vivo) in a cancer-carrying rat after intravenous administration of DA-α-CD-DO3MA-Gd or DA-γ-CD-DO3MA-Gd

A solution of DA-α-CD-DO3MA-Gd or DA-γ-CD-DO3MA-Gd was administered to male WKA rats (284-287 g, transplanted with rat hepatic cells cancer kDH-8) anesthetized with ravonal, via a cannula fixed to tail vein (0.2 mmol (Gd)/kg). The rats were fixed prone in the magnetic field of a MRI apparatus and each cancer-carrying region was imaged.

The apparatus (Omega CSI manufactured by Bruker) had a magnetic field intensity of 2.0T and, as an imaging coil, a rat Body coil was used. Imaging was carried out according to spin echo method of T1 weighed (TR/TE: 600/12 msec) under the condition of 2 mm in slice thickness, a resolution of 256 x 128.

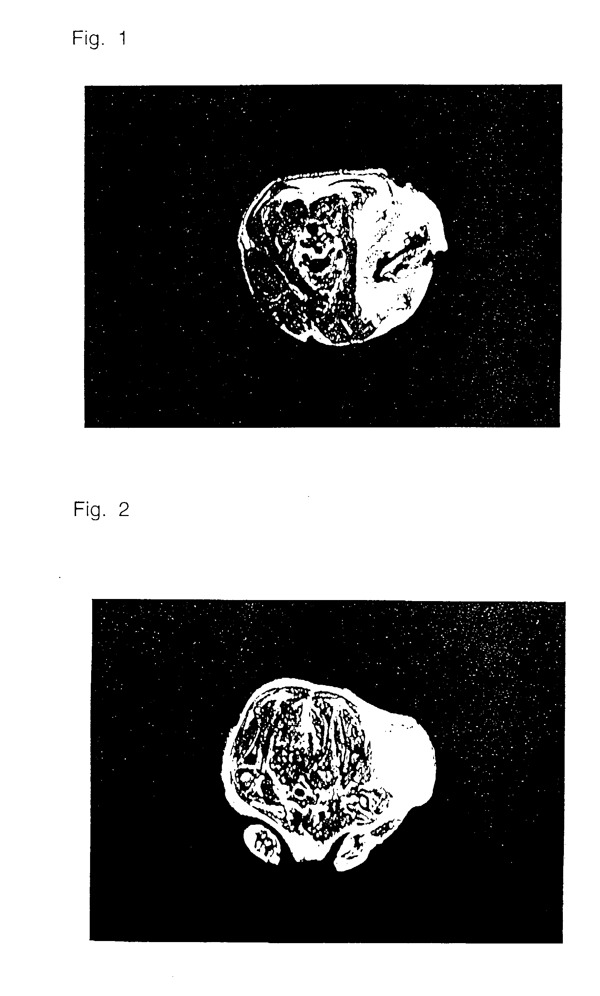

The results are shown in Figures 1 and 2.

## Claims

1. A diagnostic imaging agent which comprises a compound of (a) a complexing agent having at least one ligand chemically coupled to an aldehyde group of a dialdehyde-cyclodextrin wherein at least one constituent monosaccharide cyclodextrin is oxidation-cleaved, coordinated with (b) at least one metal ion selected from the group consisting of metal ions having the atomic number of 21-29, 31, 32, 37-39, 42-44, 49 and 56-83.

2. A diagnostic imaging agent according to claim 1, wherein at least one ligand is an diethylenetriaminepentaacetic acid derivative.

3. A diagnostic imaging agent according to claim 1, wherein at least one ligand is an 1,4,7,10-tetraazacyclododecane-1,4,7,10-tetraacetic acid derivative.

4. A diagnostic imaging agent according to claim 1, wherein at least one ligand is an 1,4,8,11-tetraazacyclotetradecane-1,4,8,11-tetraacetic acid derivative.

5. A diagnostic imaging agent according to claim 2, wherein the diethylenetriaminepentaacetic acid derivative is 1-(p-aminobenzyl)diethylenetriaminepentaacetic acid.

6. A diagnostic imaging agent according to claim 3, wherein the 1,4,7,10-tetraazacyclododecane-1,4,7,10-tetraacetic acid derivative is 1,4,7,10-tetraazacyclododecane-1-aminoethylcarbamoylmethyl-4,7,10-tris[(R,S)-methylacetic acid].

7. A diagnostic imaging agent according to any one of claims 1, 2, 3, 4, 5 or 6, wherein the metal ion is Gd, Dy, Tb, Ho, Er or Fe ion.

8. A diagnostic imaging agent according to any one of claims 1, 2, 3, 4, 5 or 6, wherein the metal ion is Bi, Pb, Tb or Os ion.

9. A diagnostic imaging agent according to any one of claims 1, 2, 3, 4, 5 or 6, wherein the metal ion is Co, Cu, Ga, Ge, Sr, Y, Tc, In, Sm, Gd, Yb, Re or Ir ion.

## Patentansprüche

1. Mittel zur Bilderzeugung für die Diagnose, umfassend eine Verbindung aus (a) einem Komplexbildner mit wenigstens einem Liganden, der an eine Aldehydgruppe eines Dialdehydcyclodextrins chemisch gebunden ist, wobei wenigstens ein als Bestandteil dienendes Monosaccharidcyclodextrin oxidativ gespalten ist, der (b) mit wenigstens einem Metallion koordiniert ist, das ausgewählt ist aus der Gruppe bestehend aus Metallionen mit der Ordnungszahl 21 - 29, 31, 32, 37 - 39, 42 - 44, 49 und 56 - 83.

2. Mittel zur Bilderzeugung für die Diagnose nach Anspruch 1, wobei wenigstens ein Ligand ein Diethylentriaminpentaessigsäure-Derivat ist.

3. Mittel zur Bilderzeugung für die Diagnose nach Anspruch 1, wobei wenigstens ein Ligand ein 1,4,7,10-Tetraazacyclododecan-1,4,7,10-tetraessigsäure-Derivat ist.

4. Mittel zur Bilderzeugung für die Diagnose nach Anspruch 1, wobei wenigstens ein Ligand ein 1,4,8,11-Tetraazacyclotetradecan-1,4,8,11-tetraessigsäure-Derivat ist.

5. Mittel zur Bilderzeugung für die Diagnose nach Anspruch 2, wobei das Diethylentriaminpentaessigsäure-Derivat 1-(p-Aminobenzyl)diethylentriaminpentaessigsäure ist.

6. Mittel zur Bilderzeugung für die Diagnose nach Anspruch 3, wobei das 1,4,7,10-Tetraazacyclododecan-1,4,7,10-tetraessigsäure-Derivat 1,4,7,10-Tetraazacyclododecan-1-aminoethylcarbamoyl-4,7,10-tris[(R,S)-methylessigsäure] ist.

7. Mittel zur Bilderzeugung für die Diagnose nach einem der Ansprüche 1, 2, 3, 4, 5 oder 6, wobei das Metallion ein Gd-, Dy-, Tb-, Ho-, Er-oder Fe-Ion ist.

8. Mittel zur Bilderzeugung für die Diagnose nach einem der Ansprüche 1, 2, 3, 4, 5 oder 6, wobei das Metallion ein Bi-, Pb-, Tb- oder Os-Ion ist.

9. Mittel zur Bilderzeugung für die Diagnose nach einem der Ansprüche 1, 2, 3, 4, 5 oder 6, wobei das Metallion ein Co-, Cu-, Ga-, Ge-, Sr-, Y-, Tc-, In-, Sm-, Gd-, Yb-, Re- oder Ir-Ion ist.

## Revendications

1. Agent de diagnostic formateur d'images qui comprend un composé de (a) un agent complexant ayant au moins un ligand couplé chimiquement à un groupe aldéhyde d'une cyclodextrine-dialdéhyde dans laquelle au moins un constituant cyclodextrine monosaccharide est clivé par oxydation, coordonné avec (b) au moins un ion métallique choisi dans le groupe constitué par les ions métalliques ayant un nombre atomique de 21-29, 31, 32, 37-39, 42-44, 49 et 56-83.

2. Agent de diagnostic formateur d'images selon la revendication 1 dans lequel au moins un ligand est un dérivé d'acide diéthylènetriaminepentaacétique.

3. Agent de diagnostic formateur d'images selon la revendication 1 dans lequel au moins un ligand est un dérivé d'acide 1,4,7,10-tétraazacyclododécane-1,4,7,10-tétraacétique.

4. Agent de diagnostic formateur d'images selon la revendication 1 dans lequel au moins un ligand est un dérivé d'acide 1,4,8,11-tétraazacyclotétradécane-1,4,8,11-tétraacétique.

5. Agent de diagnostic formateur d'images selon la revendication 2 dans lequel le dérivé d'acide diéthylènetriaminepentaacétique est l'acide 1-(p-aminobenzyl)-diéthylènetriaminepentaacétique.

6. Agent de diagnostic formateur d'images selon la revendication 3 dans lequel le dérivé d'acide 1,4,7,10-tétraazacyclododécane-1,4,7,10-tétraacétique est l'acide 1,4,7,10-tétraazacyclododécane-1-aminoéthylcarbamoylméthyl-4,7,10-tris[(R,S)-méthylacétique].

7. Agent de diagnostic formateur d'images selon l'une quelconque des revendications 1, 2, 3, 4, 5 ou 6 dans lequel l'ion métallique est un ion Gd, Dy, Tb, Ho, Er ou Fe.

8. Agent de diagnostic formateur d'images selon l'une quelconque des revendications 1, 2, 3, 4, 5 ou 6 dans lequel l'ion métallique est un ion Bi, Pb, Tb ou Os.

9. Agent de diagnostic formateur d'images selon l'une quelconque des revendications 1, 2, 3, 4, 5 ou 6 dans lequel l'ion métallique est un ion Co, Cu, Ga, Ge, Sr, Y, Tc, In, Sm, Gd, Yb, Re ou Ir.
